Europäisches Patentamt

European Patent Office

Office européen des brevets

(19)

(11) Veröffentlichungsnummer : **0 044 514**
**B1**

(12) **EUROPÄISCHE PATENTSCHRIFT**

(45) Veröffentlichungstag der Patentschrift :
13.07.83

(21) Anmeldenummer : 81105531.8

(22) Anmeldetag : 15.07.81

(51) Int. Cl.³ : **C 07 C 33/22**, C 07 C 69/06,
C 07 C 29/04, C 07 C 67/04

(54) **Verfahren zur Herstellung von Dimethylbenzylcarbinol und Dimethylbenzylcarbinylformiat.**

(30) Priorität : 19.07.80 DE 3027478

(43) Veröffentlichungstag der Anmeldung :
27.01.82 Patentblatt 82/04

(45) Bekanntmachung des Hinweises auf die Patenterteilung : 13.07.83 Patentblatt 83/28

(84) Benannte Vertragsstaaten :
CH DE FR GB IT LI NL

(56) Entgegenhaltungen :
DE A 2 430 470
DE A 2 726 064
US A 2 485 125

INDUSTRIAL & ENGINEERING CHEMISTRY Product Research & Development, Band 2, Nr. 2, Juni
1963 Washington, US L. O. : RAETHER et al. :
« Acid-Olefin Esterification », Seiten 133-138

(73) Patentinhaber : **Ruhrchemie Aktiengesellschaft
Bruchstrasse 219
D-4200 Oberhausen 13 (DE)**

(72) Erfinder : **Bernhagen, Wolfgang, Dr. Dipl.-Chem.
D-4330 Mülheim/Ruhr (DE)**
Erfinder : **Springer, Helmut
Drostenkampstrasse 24
D-4200 Oberhausen 13 (DE)**

(74) Vertreter : **Reichelt, Karl-Heinz, Dr.
m. Br. Ruhrchemie Aktiengesellschaft Abt. PLD Postfach 13 01 60
D-4200 Oberhausen 13 (DE)**

## Verfahren zur Herstellung von Dimethylbenzylcarbinol und Dimethylbenzylcarbinylformiat

Die vorliegende Erfindung betrifft ein neues Verfahren zur Herstellung von Dimethylbenzylcarbinol und Dimethylbenzylcarbinylformiat. Dimethylbenzylcarbinol ist aufgrund seines mild-blumigen, etwas grünen Geruches, der an Holunder und Flieder erinnert, ein sehr wertvoller Grundstoff für die Riechstoffindustrie. Aus dem Dimethylbenzylcarbinylformiat können durch Umesterung höhere Ester gewonnen werden, die ebenfalls in der Parfümerie Anwendung finden.

Zur Herstellung von Dimethylbenzylcarbinol sind eine Reihe von Verfahren bekannt. So läßt man nach einer Arbeitsweise Aceton mit einem Benzylmagnesiumhalogenid reagieren und hydrolysiert die gebildete Additionsverbindung (veröffentlichte JA-Patentanmeldung 1974-30 333). Eine andere Synthese geht von Tetrabenzyltitan aus, das mit Aceton umgesetzt wird (J. Causse et al., Helv. Chim. Acta 55 (1972) 5, 1560-66).

Diese Verfahren verlaufen über metallorganische Zwischenstufen, ihre Anwendung im großtechnischen Maßstab ist daher technisch aufwendig und wirtschaftlich von geringem Interesse.

Die Bildung metallorganischer Zwischenstufen wird durch die in der DE-PS 27 26 064 beschriebene Arbeitsweise vermieden. Der Prozeß geht von Neophylchlorid aus. Die Reaktion mit Natriumformiat ergibt ß,ß-Dimethylstyrol, an das Ameisensäure unter Bildung von Dimethylbenzylcarbinylformiat angelagert wird. Durch Verseifung erhält man hieraus das Carbinol. Nachteilig ist, daß die Ameisensäureanlagerung zur Erzielung eines zufriedenstellenden Umsatzes übermäßig lange Reaktionszeiten und einen extrem hohen Ameisensäureüberschuß erfordert.

Es bestand somit die Aufgabe, ein Verfahren zur Herstellung von Dimethylbenzylcarbinol bereitzustellen, das in technisch leicht durchführbaren Reaktionsschritten das gewünschte Produkt in guten Ausbeuten zugänglich macht.

Die Erfindung besteht in einem Verfahren zur Herstellung von Dimethylbenzylcarbinol und Dimethylbenzylcarbinylformiat aus 2-Methyl-1-phenyl-2-propen und Ameisensäure in Gegenwart katalytischer Mengen einer starken Mineralsäure, wobei die Umsetzung in Gegenwart eines aliphatischen Alkohols, der 1 bis 10 Kohlenstoffatome enthält, eines cycloaliphatischen oder aromatischen Alkohols, der bis zu 10 Kohlenstoffatome aufweist, vorgenommen wird, das Molverhältnis von Olefin zu Ameisensäure 1 zu 1 bis 10, das von Olefin zu Alkohol 1 zu 0,1 bis 2 und das von Olefin zu Katalysator 1 zu 0,05 bis 0,2 beträgt, als Katalysator Schwefelsäure eingesetzt wird und die Reaktion bei 5 bis 50 °C durchgeführt wird.

Aus dem Reaktionsgemisch kann einerseits durch Neutralisation der freien Ameisensäure und des Katalysators ein Gemisch aus Dimethylbenzylcarbinol und Dimethylbenzylcarbinylformiat oder andererseits durch Verseifung mit überschüssigem Alkalihydroxid ausschließlich Dimethylbenzylcarbinol gewonnen werden.

Die beanspruchte Arbeitsweise geht von 2-Methyl-1-phenyl-2-propen aus. Dieses Olefin erhält man nach bekannten Verfahren, z. B. durch Dehydratisierung von 2-Methyl-3-phenyl-1-propanol.

Die Anlagerung von Carbonsäuren an Olefine unter dem katalytischen Einfluß starker Säuren, die zur Bildung von Estern führt, ist eine bekannte Reaktion. Sie verläuft nach einem Carboniumionenmechanismus. Bei Einsatz eines verzweigten Olefins entstehen daher die Ester tertiärer Alkohole(Markownikoff-Addition). Der saure Katalysator bewirkt jedoch auch eine Stellungsisomerisierung der Kohlenstoff-Kohlenstoff-Doppelbildung. Aus dem 2-Methyl-1-phenyl-2-propen bildet sich daher leicht das wesentlich stabilere 2-Methyl-1-phenyl-1-propen. Dieses Isomere ist nun der Reihe jener verzweigten höhermolekularen Olefine zuzuordnen, die aus sterischen Gründen mit Carbonsäuren nur noch sehr langsam reagieren (vgl. L.O. Raether et al., Ind. Eng. Chem., Prod. Res. Develop. 2, 133 (1963)). Die Ergebnisse entsprechender Versuche bestätigen diese theoretischen Überlegungen.

Überraschenderweise kann die unerwünschte Isomerisierung des 2-Methyl-1-phenyl-2-propens weitgehend unterdrückt und die Carbonsäureanlagerung in hohen Ausbeuten durchgeführt werden, wenn man dem Reaktionsgemisch einen aliphatischen, cycloaliphatischen oder aromatischen Alkohol zusetzt. Die Struktur des Alkohols und seine Molekülgröße ist dabei ohne großen Einfluß, Methanol eignet sich ebenso wie Isononylalkohol oder Benzylalkohol. Andere mit Erfolg eingesetzte Alkohole sind Isopropanol, n-Butanol, Isobutanol, Amylalkohol, Cyclohexanol und 2-Ethylhexanol. Vorzugsweise verwendet man Alkohole mit 1 bis 10 Kohlenstoffatomen, die keine weiteren Substituenten enthalten.

Die Molmengen, in denen Olefin, Ameisensäure, Katalysator und Alkohol eingesetzt werden, richten sich nach der Arbeitstemperatur, die 5 bis 50 °C, vorzugsweise 10-30 °C, beträgt. Je Mol Olefin wendet man 1 bis 10 Mol, vorzugsweise 2-5 Mol Ameisensäure, 0,1 bis 2 Mol, vorzugsweise 0,2 bis 1 Mol Alkohol und 0,05 bis 0,2 Mol, vorzugsweise 0,1 Mol Katalysator zum Einsatz an. Als Katalysator hat sich insbesondere Schwefelsäure bewährt.

Nach einer zweckmäßigen Ausführungsform des erfindungsgemäßen Verfahrens fügt man zum Gemisch aus Ameisensäure und Katalysator zunächst den Alkohol zu. In exothermer Reaktion bildet sich dabei das entsprechende Alkyl-, Cycloalkyl- oder Arylformiat. Danach stellt man die gewünschte Arbeitstemperatur ein und setzt unter intensiver Durchmischung das Olefin zu. Das anfangs heterogene, später homogene Gemisch wird nach Beendigung der Olefinzugabe zwischen 15 Minuten und 4 Stunden gerührt. Die Reaktionszeit ist hierbei von der eingestellten Temperatur und der angewandten Alkoholmenge abhängig, so daß sie im Einzelfall empirisch bestimmt werden muß. Höhere Temperaturen und niedrige Alkoholkonzentration erfordern kürzere Reaktionszeiten als niedere Temperatur und höhere Alkoholkonzentration. Ein typischer Ansatz wird beispielsweise unter Anwendung von 5 Mol Ameisensäure, 0,1 Mol Schwefelsäure und 0,5 Mol Alkohol je Mol Olefin bei 10 °C und zweistündiger Reaktionszeit durchgeführt.

Nach Abschluß der Reaktion stehen zur Aufarbeitung des Rohproduktes zwei Wege offen. Einmal kann das im Reaktionsprodukt enthaltene Dimethylbenzylcarbinylformiat unmittelbar, d. h. ohne Vorbehandlung oder Abtrennung mit überschüssigem Alkalihydroxid verseift werden, wobei mittels fraktionierter Destillation als Wertprodukt ausschließlich Dimethylbenzylcarbinol aus der organischen Phase gewonnen wird.

Es ist jedoch auch möglich, das Reaktionsgemisch nur zu neutralisieren. Dann erhält man neben dem in Gemisch vorhandenen Dimethylbenzylcarbinol auch das bei der Synthese gebildete Dimethylbenzylcarbinylformiat.

Die Abtrennung der beiden Produkte aus der organischen Phase des Reaktionsgemisches erfolgt durch fraktionierte Destillation. Das Formiat ist eine äußerst interessante Verbindung zur Herstellung der höheren Ester wie des Acetates, Propionates, Isobutyrates usw.

Vorteilhaft wird diese Reaktion in Art einer Umesterung unter Anwendung der entsprechenden Methylester durchgeführt.

In den folgenden Beispielen ist die Erfindung näher beschrieben.

Beispiel 1

In einem Rundkolben gibt man zu 469 g 98 %-iger Ameisensäure und 130 g n-Butanol unter Rühren nacheinander 20 g konzentrierte Schwefelsäure und 264 g 2-Methyl-1-phenyl-2-propen, wobei die Temperatur des Gemisches 30 °C nicht übersteigt. Nach Beendigung der Zugabe rührt man noch 2 Stunden bei 30 °C. Anschließend wird das Rohprodukt mit 1 600 g 30 %iger Natronlauge versetzt und kurzzeitig auf 80 °C erwärmt. Nach gaschromatographischer Analyse enthält die organische Phase

35,6 % n-Butanol
5,4 % 2-Methyl-1-phenyl-2-propen
6,0 % 2-Methyl-1-phenyl-1-propen
50,7 % Dimethylbenzylcarbinol
0,4 % Dimethylbenzylcarbinylformiat
1,9 % Sonstige Komponenten

Durch fraktionierte Destillation können aus dem Ansatz 212 g Dimethylbenzylcarbinol (102 °C/13 mbar) gewonnen werden. Dies entspricht einer Ausbeute von 71 %, bezogen auf eingesetztes Olefin.

Beispiel 2

Man arbeitet wie im Beispiel 1 beschrieben, abweichend davon jedoch bei 10 °C und unter Einsatz von 74 g n-Butanol. Nach Destillation erhält man 228 g Dimethylbenzylcarbinol. Dies entspricht einer Ausbeute von 76 %, bezogen auf eingesetztes Olefin.

Beispiel 3

Man arbeitet wie in Beispiel 1 beschrieben, ersetzt jedoch n-Butanol durch 64 g Methanol und vermindert die Reaktionszeit von 120 auf 90 Minuten. 218 g (73 % Ausbeute) Dimethylbenzylcarbinol fallen dabei an.

Beispiel 4

Man arbeitet wie in Beispiel 1 beschrieben, setzt jedoch nur 188 g 98 %ige Ameisensäure, 74 g n-Butanol sowie 700 g 30 %ige Natronlauge ein. Nach Destillation fallen 185 g (61 % Ausbeute) Dimethylbenzylcarbinol an.

Beispiel 5

In einem Rundkolben gibt man zu 234 g 98 %-iger Ameisensäure und 37 g n-Butanol unter Rühren nacheinander 10 g konzentrierte Schwefelsäure und 132 g 2-Methyl-1-phenyl-2-propen, wobei die Temperatur des Gemisches durch Kühlung bei 10 °C gehalten wird. Nach 2 Stunden Reaktion bei 20 °C versetzt man das Reaktionsgemisch mit 600 g 30 %iger Natronlauge. Die Innentemperatur wird durch Kühlung auf maximal 25 °C begrenzt. Die organische Phase enthält nach gaschromatographischer Analyse

2,3 % n-Butanol
23,8 % n-Butylformiat
5,0 % 2-Methyl-1-phenyl-propen-2
6,6 % 2-Methyl-1-phenyl-propen-1
24,5 % Dimethylbenzylcarbinol
35,9 % Dimethylbenzylcarbinylformiat
1,9 % Sonstige Komponenten

Durch fraktionierte Destillation erhält man 54 g Dimethylbenzylcarbinol (36 % Ausbeute) und 80 g Dimethylbenzylcarbinylformiat (45 % Ausbeute bezogen auf Olefin).

Abweichend von dem erfindungsgemäßen Verfahren wurden die folgenden Vergleichsbeispiele ohne Zusatz eines Alkohols durchgeführt.

Vergleichsbeispiel 1

In einem Rundkolben werden 469 g 98 %ige Ameisensäure, 20 g $H_2SO_4$ und 264 g 2-Methyl-1-phenyl-2-propen unter Rühren 2 Stunden auf 30 °C erwärmt. Nach Zugabe von 1 600 g 30 %iger Natronlauge und Phasentrennung weist die organische Phase folgende Zusammensetzung auf :

2,8 % 2-Methyl-1-phenyl-2-propen
66,7 % 2-Methyl-1-phenyl-1-propen
21,9 % Dimethylbenzylcarbinol
8,6 % Sonstige Komponenten

Durch fraktionierte Destillation werden 60 g Dimethylbenzylcarbinol (entsprechend 20 % Ausbeute) isoliert.

Vergleichsbeispiel 2

Man arbeitet wie im Vergleichsbeispiel 1, wählt jedoch eine Reaktionstemperatur von 10 °C. Die Ausbeute an Dimethylbenzylcarbinol beträgt < 93 g (entsprechend 31 % Ausbeute bezogen auf Olefin).

**Anspruch**

Verfahren zur Herstellung von Dimethylbenzylcarbinol und Dimethylbenzylcarbinylformiat aus 2-Methyl-1-phenyl-2-propen und Ameisensäure in Gegenwart katalytischer Mengen einer starken Mineralsäure, dadurch gekennzeichnet, daß die Umsetzung in Gegenwart eines aliphatischen Alkohols, der 1 bis 10 Kohlenstoffatome enthält, oder eines cycloaliphatischen oder aromatischen Alkohols, der bis zu 10 Kohlenstoffatome aufweist, vorgenommen wird, das Molverhältnis von Olefin zu Ameisensäure 1 : 1 bis 10, das von Olefin zu Alkohol 1 : 0,1 bis 2 und das von Olefin zu Katalysator 1 : 0,05 bis 0,2 beträgt, als Katalysator Schwefelsäure eingesetzt und die Reaktion bei 5 bis 50 °C durchgeführt wird.

**Claim**

Process for preparing dimethylbenzyl carbinol and dimethylbenzyl carbinyl formate from 2-methyl-1-phenyl-2-propene and formic acid in the presence of catalytic amounts of a strong mineral acid, characterised in that the conversion is carried out in the presence of an aliphatic alcohol containing 1 to 10 carbon atoms, a cycloaliphatic or aromatic alcohol, which has up to 10 carbon atoms, the mole ratio of olefin to formic acid is 1 : 1 to 10, that of olefin to formic acid is 1 : 0.05 to 0.2 and that of olefin to catalyst 1 : 0.05 to 0.2, sulfuric acid is used as a catalyst and the reaction takes place at 5 to 50 °C.

**Revendication**

Procédé de préparation de diméthylbenzylcarbinol et de formiate de diméthylbenzylcarbinyle à partir de 2-méthyl-1-phényl-2-propène et d'acide formique en présence de quantités catalytiques d'un acide minéral fort, procédé caractérisé en ce qu'on effectue la réaction en présence d'un alcool aliphatique qui contient 1 à 10 atomes de carbone, d'un alcool cycloaliphatique ou aromatique qui présente jusqu'à 10 atomes de carbone ; le rapport molaire de l'oléfine à l'acide formique est compris entre 1 : 1 et 1 : 10, celui de l'oléfine à l'alcool entre 1 : 0,1 et 1 : 2 et celui de l'oléfine au catalyseur entre 1 : 0,05 et 1 : 0,2 ; on utilise comme catalyseur l'acide sulfurique, et l'on effectue la réaction entre 5 et 50 °C.